# EUROPÄISCHE PATENTANMELDUNG

(11) **EP 0 976 365 A1**
(43) Veröffentlichungstag der Anmeldung: **02.02.2000**
(21) Anmeldenummer: 98810717.3
(22) Anmeldetag: 27.07.1998
(51) Int. Cl.: A61B 17/72

(54) **Retrograder Tibianagel**

(71) Anmelder: OSTEO AG, 2545 Selzach (CH)
(72) Erfinder: Bühren, Volker, 82418 Murnau (DE); Hofmann, Gunther O., 86938 Schondorf/Ammersee (DE); Gonschorek, Oliver, 82419 Murnau (DE); Wahl, Thomas, 2543 Lengnau (CH); Sutter, Lukas, 2545 Selzach (CH); Bernard, Andreas, 2554 Meinisberg (CH)
(74) Vertreter: Münch, Otto, Dipl.-Ing.

(57) **Zusammenfassung**

Der Tibianagel besteht aus einem Rohr mit einer durchgehenden Längsbohrung (7) und hat einen proximalen Verankerungsteil (2) mit mehreren Querbohrungen (12, 13), einen anschliessenden, in anterior-posteriorer Richtung biegeelastischen Verbindungsteil (3), einen daran anschliessenden Schaft (4) und einen distalen Verankerungsteil (5). Der Verankerungsteil (5) ist gegenüber dem Schaft (4) abgebogen und hat ein Langloch (32), durch welches eine Verriegelungsschraube gesteckt werden kann. Mittels einer in eine Längs-Gewindebohrung eingesetzten Kompressionsschraube kann die Tibia komprimiert werden. Der Tibianagel ermöglicht die retrograde Implantation, was bisher für die Tibia nicht für möglich gehalten wurde.

## Beschreibung

Die retrograden Implantationen von Marknägeln in Femur und Humerus sind derzeit bereits routinemässige Osteosynthese-Verfahren. Dagegen erschien bislang die Implantation eines Marknagels in retrograder Richtung in die Tibia aufgrund anatomischer und implantat-technischer Überlegungen als unmöglich. Insbesondere bei Brüchen im proximalen Bereich der Tibia wurden deshalb bisher z.B. Osteosynthese-Platten verwendet.

Der Erfindung liegt die Aufgabe zugrunde, ein Implantat zu entwickeln, das einen höheren Patientenkomfort ermöglicht. Diese Aufgabe wird durch die Merkmalskombination der Ansprüche gelöst.

Nachfolgend wird ein Ausführungsbeispiel der Erfindung anhand der Zeichnungen erläutert. Darin zeigt:
- Figur 1: eine Seitenansicht eines Tibianagels,
- Figur 2: eine Draufsicht auf den Nagel,
- Figuren 3 bis 7: Schnitte längs den Linien III-III bis VII-VII in Figur 1, und
- Figur 8: eine Stirnansicht in Richtung des Pfeils VIII in Figur 1.

Der intramedulläre Tibianagel 1 wird aus einem biologisch verträglichen Metall hergestellt. Er dient der Knochenbruchbehandlung im proximalen Teil der Tibia und zur temporären Stabilisierung von hohen Tibia-Osteotomien. Der Nagel 1 besteht aus vier Abschnitten: einem biegesteifen proximalsten Teil 2, einem proximalen, in anterior-posteriorer Richtung biegeelastischen Teil 3, einem medialen Schaft 4 und einem distalen, aufgeweiteten Teil 5 zur Aufnahme der distalen Verriegelungsschrauben 6. Der ganze Nagel ist kanuliert ausgeführt (Bohrung 7), um die Einführung mittels eines Zieldrahtes oder Führungsdrahtes zu ermöglichen. In der Regel ist er rotationssymmetrisch ausgebildet. Die Durchmesser der einzelnen Teile 2-5 variieren, sind typischerweise für den proximalen Teil 2 etwa 9 mm, beim Schaft 4 etwa 7 mm und im distalen Teil 5 etwa 10 mm. Der Nagel 1 wird in einem Längenspektrum von etwa 200 bis 400 mm hergestellt, wobei im wesentlichen die Länge des Schaftes 4 variiert wird.

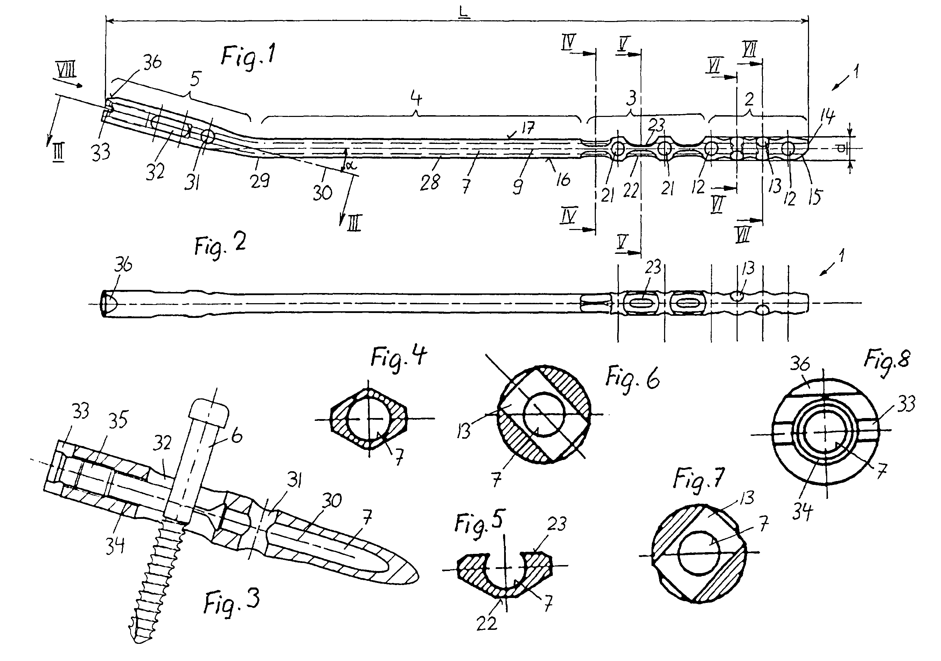

Der proximalste biegesteife Teil 2 weist mindestens zwei winkelversetzte Schraubenlöcher 12, 13 für proximale Verriegelungsschrauben auf, bei denen im Unterschied zu den Schrauben 6 nach Fig. 3 das Gewinde bis zum Schraubenkopf geschnitten ist. Mindestens ein Loch 12 liegt in medio-lateraler Richtung. Im Beispiel sind vier Löcher 12, 13 dargestellt, wobei das proximalste und das distalste in medio-lateraler Richtung liegen, und die zwei Löcher 13 dazwischen jeweils um einen Winkel um die axiale Richtung des Nagels gedreht sind, im dargestellten Beispiel um etwa 45°. Die Anordnung der Löcher 12, 13 erlaubt es, mehrere Fragmente im Bereich des Tibiaplateaus an ihrer Position zu fixieren. Im biegesteifen Teil 2 ist der Querschnitt über die ganze Länge etwa gleich. Die Biegesteifigkeit ist auf der Höhe der Löcher 12, 13 am grössten, während die Verbindungsteile zwischen den Löchern 12, 13 eine allfällige Verformung aufnehmen. Dieser Teil 2 ist um die Längsachse 9 des Nagels 1 rotationssymmetrisch ausgestaltet. Die proximale Spitze 14 hat eine kufenförmige Anfräsung 15 mit einem grossen Radius an der posterioren Seite 16, was eine erleichterte Einbringung ermöglicht. Der Radius der Anfräsung 15 ist grösser als der halbe Durchmesser d des Teils 2.

Der axial anschliessende zweite Teil 3 weist zwei Löcher 21 in medio-lateraler Richtung auf, wobei die benachbarten Abschnitte tangentiale Anfräsungen 22, 23 auf der anterioren 17 und der posterioren 16 Seite aufweisen, die dem Nagel 1 eine grössere Flexibilität in diesen Richtungen geben. Dies ist besonders wichtig, weil der Nagel 1 von der anterioren Seite 17 der Tibia eingebracht wird und dabei quasi um die Ecke gebracht werden muss. Auf der anterioren Seite 17 in diesem biegeelastischen Teil 3 sind die Anfrasungen 23 tiefer und schneiden die Bohrung 7 (Fig. 5), weil die Biegung beim Einsetzen in anteriorer Richtung ist. Die Biegeelastizität ist in anterior-posteriorer Richtung bedeutend höher als in Richtung senkrecht dazu. In der Gegend der Löcher 21 ist der Teil 3 kreiszylindrisch. Diese Bereiche 24 dienen als Stützen und verhindern ein Ausknicken des Teils 3 unter Druck.

Der an den biegeelastischen Teil 3 anschliessende Schaft 4 des Nagels 1 besteht aus einem Rohr 28, welches den Teil 3 und den distalen Verriegelungsteil 5 miteinander verbindet. Dieses Rohr 28 weist typischerweise einen Durchmesser zwischen 5 und 15 mm auf. Im dargestellten Beispiel wurde der Durchmesser so gewählt, dass das Rohr 28 bei genügender Festigkeit so weich wie möglich ausgestaltet wurde. Am distalen Ende dieses Rohres 28 befindet sich eine Krümmung 29, welche überleitet in den distalen Verriegelungsteil 5. Der Winkel α zwischen der Achse 9 im Rohr 28 und der Achse 30 im distalen Teil 5 kann zwischen 5° und 30° betragen. Im dargestellten Beispiel ist er 15°. Im weiteren vergrössert sich der Querschnitt des Rohrs 28 in der Krümmung 29, so dass ein stetiger Übergang vom kleineren Durchmesser des Rohres 28 zum grösseren Durchmesser des distalen Verriegelungsteils 5 stattfindet.

Dieser distale Verriegelungsteil 5 weist mindestens zwei Querbohrungen 31, 32 auf, wobei die distale Bohrung 32 als Langloch in axialer Richtung ausgeführt ist. Diese Bohrungen 31, 32 dienen der Aufnahme der distalen Verriegelungsbolzen 6, die proximale Bohrung 31 für statische Verriegelungsbolzen, das Langloch 32 für dynamische oder Kompressions-Verriegelungsbolzen 6.

Am distalen Ende befindet sich eine ca. 3 bis 5 mm tiefe Nut 33, die radial durch den Durchmesser des Nagels 1 führt. Die axiale Aufbohrung des Nagels 1 ist hier am grössten. An diese Nut 33 anschliessend beginnt ein Innengewinde 34 mit geringerem Innendurchmesser in axialer proximaler Richtung, welches kurz vor der distalsten Querbohrung 32 endet. Die distale Nut 33 und das Innengewinde 34 dienen als Schnittstelle zum Zielgerät bei der Einbringung, als Andockstelle bei der Extraktion und der Aufnahme einer Kompressions- oder Verschlussschraube 35.

Die Längsbohrung des distalen Verriegelungsteils weist zudem einen grösseren Durchmesser als die Kanulation 7 des restlichen Nagels 1 auf, damit eine Kompressionsschraube 35 aufgenommen werden kann, die senkrecht auf den Bolzen 6 in der distalen Bohrung 32 drückt. Durch Eindrehen der Schraube 35 kann somit eine Kompression der Tibia erreicht werden. Am distalen, anterioren Ende des Nagels 1 befindet sich zudem eine Anfasung 35, welche Weichteilschäden vermeiden soll, falls der Nagel infolge Kompression etwas aus dem Knochen austreten sollte.

Zur Operation wird der Patient auf dem Rücken gelagert, mit Blutsperre am Oberschenkel und frei beweglich abgedecktem Bein. Der Zugang erfolgt über eine Längsinzision an der Vorderseite des distalen Unterschenkels und des oberen Sprunggelenkes mit 5 bis 7 cm Länge. Nach der Spaltung von Subcutis und Faszia werden die Retinacula der Extensoren in Längsrichtung gespalten. Der Musculus extensor halluzis longus wird nach medial retrahiert, die Tibialis anterior-Gefässe und der Nervus peronaeus profundus werden nach lateral mit einem Venenhaken retrahiert. Der anteriore Cortex der distalen Tibia wird subperiostal freigelegt. Es wird sorgfältig darauf geachtet, dass die Gelenkkapsel des oberen Sprunggelenkes nicht eröffnet wird.

Mit einem Pfriem wird der Cortex der distalen Tibia knapp über dem Ansatz der Gelenkkapsel eröffnet. Ein Führungsdraht mit leicht angebogener Spitze wird in den Markraum eingeführt und über die Fraktur bzw. die Osteotomie hinweg in die proximale Tibia bis in die Emminentia intercondylaris hochgeschoben. Der Markkanal wird mit flexiblen Bohrwellen in retrograder Richtung eröffnet. Der Aufbohrvorgang beginnt bei einem Durchmesser der Bohrköpfe von 6 mm, setzt sich in 0,5 mm Schritten fort und endet bei einem Durchmesser von 1 mm grösser als der ausgesuchte Marknagel 1.

Der Isthmus der Tibia bleibt dabei die Region für eine feste intramedulläre Fixierung des Marknagels 1. In den meisten Fällen kann der ausgewählte Marknagel 1 per Hand vorgeschoben werden. Wenn notwendig können leichte Schläge mit dem Hammer das Einsetzen des Nagels 1 erleichtern. Falls der Widerstand beim Einsetzen zu gross wird, wird ein kleinerer Nageldurchmesser gewählt. Der Nagel 1 sollte mit seinem Ende (Anfasung 36) etwas tiefer eingesetzt werden als die Oberfläche der ventralen Tibiakante. Nach Einbringen der Kompressionsschraube 35 und Ausüben der Kompression wird der Nagel 1 dann bündig mit der Knochenoberfläche abschliessen.

Die proximale Verriegelung im Tibiakopf erfolgt in FreihandTechnik durch den Tibiakopf. Dabei verwendet man vorzugsweise selbstschneidende 5 mm Schrauben. Die Verriegelungsschrauben im Tibiakopf werden vorzugsweise von medial nach lateral und von ventral nach dorsal eingesetzt. Die distalen Verriegelungsschrauben 6 werden mit Hilfe eines Zielgerätes eingesetzt.

Der beschriebene Tibianagel hat folgende Vorteile:
- Er ermöglicht ein Einsetzen in retrograder Richtung und kommt speziell bei ganz hohen Tibiakopfbrüchen zum Einsatz, wo bislang eine Marknagel-Implantation gänzlich unmöglich war.
- Als intramedullärer Kraftträger ist er früh belastbar und weitaus stabiler als herkömmliche Fixationsarten. Dies ermöglicht eine bessere physiotherapeutische Nachbehandlung des Patienten und eine rasche Genesung.
- Der Tibianagel ermöglicht eine minimal-invasive Operations-technik und eine einfache Explantation.
- Er verbessert den Patientenkomfort.
- Die periostale Blutversorgung wird durch den Tibianagel im Markraum nicht beeinträchtigt.
- Neuronale Läsionen werden vermieden.

Der beschriebene Tibianagel eignet sich vor allem für proximale Tibiafrakturen, Umstellungs-Osteotomien, Callus-Distraktionen, Segmenttransfer, Kniegelenktransplantationen usw..

## Patentansprüche

1. Retrograder Tibianagel, umfassend einen ersten Verankerungsteil (2) mit mindestens einer Querbohrung (12, 13) zur Verankerung mittels einer Verriegelungsschraube (6), einen an den ersten Verankerungsteil (2) anschliessenden biegeelastischen Verbindungsteil (3), einen daran anschliessenden Schaft (4) und einen unter einem Winkel (α) an den Schaft (4) anschliessenden zweiten Verankerungsteil (5), der mindestens eine weitere Querbohrung (31, 32) aufweist, wobei die Biegeelastizität des Verbindungsteils (3) in der die Achsen (9, 30) des Schaftes (4) und des zweiten Verankerungsteils (5) enthaltenden ersten Ebene vorzugsweise grösser ist als in einer die Achse (9) des Schaftes (4) enthaltenden, zur ersten Ebene senkrechten zweiten Ebene.

2. Tibianagel nach Anspruch 1, wobei er eine durchgehende Längsbohrung (7) aufweist.

3. Tibianagel nach Anspruch 1 oder 2, wobei er am freien Ende des ersten Verankerungsteils (2) auf der dem zweiten Verankerungsteil (5) bezüglich der Achse (7) des ersten Verankerungsteils (2) diametral gegenüberliegenden Seite eine kufenartige Anfräsung (15) aufweist.

4. Tibianagel nach einem der Ansprüche 1 bis 3, wobei der erste Verankerungsteil (2) mehrere Querbohrungen (12, 13) in unterschiedlicher Richtung aufweist, und wobei mindestens eine der Querbohrungen (12) senkrecht zur ersten Ebene verläuft.

5. Tibianagel nach einem der Ansprüche 1 bis 4, wobei der zweite Verankerungsteil (5) ein Langloch (32) aufweist sowie eine Gewinde-Längsbohrung (34) zum Einsetzen einer Kompressionsschraube (35), und wobei vorzugsweise der zweite Verankerungsteil (5) zusätzlich eine zylindrische Querbohrung (31) aufweist.

6. Tibianagel nach einem der Ansprüche 1 bis 5, wobei der Winkel (α) 5° bis 30°, vorzugsweise etwa 15° beträgt.

7. Tibianagel nach einem der Ansprüche 1 bis 6, wobei der Verbindungsteil (3) Anfräsungen (22, 23) parallel zur zweiten Ebene aufweist, und wobei vorzugsweise die Anfräsungen (23) von der einen, insbesondere der anterioren Seite her tiefer sind als von der anderen Seite und die tieferen Anfräsungen (23) die Längsbohrung (7) schneiden.

8. Tibianagel nach einem der Ansprüche 1 bis 7, wobei der Verbindungsteil (3) durch Stützkörper (24) unterteilt ist, die annähernd denselben Aussendurchmesser haben wie der erste Verankerungsteil (2), und wobei die Stützkörper (24) vorzugsweise weitere Querbohrungen (21) aufweisen.

9. Tibianagel nach einem der Ansprüche 1 bis 8, wobei der erste Verankerungsteil (2) zwischen den Querbohrungen (12, 13) Einschnürungen aufweist.

10. Tibianagel nach einem der Ansprüche 1 bis 9, wobei er am freien Ende des zweiten Verankerungsteils (5) einseitig eine Anfasung (36) aufweist.
